# EUROPEAN PATENT SPECIFICATION

(11) **EP 3 271 450 B1**
(45) Date of publication and mention of the grant of the patent: **06.11.2019**
(21) Application number: 16723511.8
(22) Date of filing: 18.03.2016
(51) Int. Cl.: C12M 1/06

(54) **METHOD AND BIOREACTOR FOR PRODUCING A CULTURE OF MICROORGANISMS**
VERFAHREN UND BIOREAKTOR ZUR HERSTELLUNG EINER MIKROORGANISMENKULTUR
PROCÉDÉ ET BIORÉACTEUR POUR LA PRODUCTION D'UNE CULTURE DE MICRO-ORGANISMES

(30) Priority: 19.03.2015 IT PG20150016
(43) Date of publication of application: 24.01.2018
(73) Proprietor: Labruzzo, Pietro, 06083 Bastia Umbra (PG) (IT)
(72) Inventor: Labruzzo, Pietro, 06083 Bastia Umbra (PG) (IT)
(74) Representative: Ercolani, Simone Pietro
(86) International application number: PCT/IB2016/051542
(87) International publication number: WO 2016/147156

(56) References cited:
- CA-A1- 2 760 882
- KR-A- 20130 082 341
- US-A1- 2014 038 257
- SCHLAFER O ET AL: "Improvement of biological activity by low energy ultrasound assisted bioreactors", ULTRASONICS, IPC SCIENCE AND TECHNOLOGY PRESS LTD. GUILDFORD, GB, vol. 38, no. 1-8, 1 March 2000 (2000-03-01), pages 711-716, XP027331276, ISSN: 0041-624X [retrieved on 2000-03-01]
- CHISTI Y: "Sonobioreactors: using ultrasound for enhanced microbial productivity", TRENDS IN BIOTECHNOLOGY, ELSEVIER PUBLICATIONS, CAMBRIDGE, GB, vol. 21, no. 2, 1 February 2003 (2003-02-01), pages 89-93, XP004405628, ISSN: 0167-7799, DOI: 10.1016/S0167-7799(02)00033-1
- RODRIGUEZ-MOLARES ALFONSO ET AL: "Determination of biomass concentration by measurement of ultrasonic attenuation", APPLIED ACOUSTICS, vol. 81, 20 March 2014 (2014-03-20), pages 26-30, XP028844641, ISSN: 0003-682X, DOI: 10.1016/J.APACOUST.2014.02.008

## Description

### Field of the Invention

The present invention relates to a method and a bioreactor for producing a culture of microorganisms, such as for example microalgae, cyanobacteria, diatoms, yeasts, etc.

### Known previous art

Bioreactors are devices allowing the stand-alone growth (without the continuous intervention of an operator) of cells or tissues in a given culture medium (generally liquid).

Effects and diffusion in the air of sounds produced by natural events, by other forms of acoustic pollution, climate events and animal interactions perceived by the vegetable world in the form of mechanical transductions, are known.

Bioreactors are known in which a culture of microorganisms placed within the same is irradiated through sound waves produced by a source emitting in the domain of human audible sound (100 Hz - 20 kHz). However, such bioreactors need sound waves having high intensity not being anyway able to significantly increase the growth of microorganisms in order to fulfill the market demands at least from the economical point of view.

### Summary of the invention

Object of the present invention is to provide a method for producing a culture of microorganisms able to accelerate the culture growth then obtaining higher growth rates than the methods of the known art.

Further object of the present invention is to provide a bioreactor for producing a culture of microorganisms easy to manufacture and that allows positively increasing the kinetics of microorganism growth in order to produce a culture of organisms more rapidly than known bioreactors.

These and additional objects are solved by the present invention by a method for producing a culture of microorganisms according to claim 1 and the respective dependent claims, and a bioreactor according to claim 8 and the respective dependent claims.

In particular, the method for producing a culture of microorganisms is carried out by means of a bioreactor comprising at least one cylindrical container and shaking and mixing means to shake and mix said culture of microorganisms contained in said container.

The method comprises the step of a) shaking and mixing said microorganisms through the shaking and mixing means, and the step of b) irradiating the culture of microorganisms with a sound wave.

According to an aspect of the present invention, during the step b) the culture is irradiated with a sound wave whose frequency changes over time as a sawtooth function. The frequency of the sound wave changes in a range comprised between 50 Hz and 8 kHz, preferably between 100 Hz and 6 kHz.

Thereby the obtained sound wave (termed in jargon "sweep") has a frequency changing over time linearly between two values taken in the range comprised between 50 Hz and 8 kHz, preferably between 100 Hz and 6 kHz. For example, the sawtooth function can be obtained from a start time t₀, in which the frequency of the sound wave is equal for example to 100 Hz, after a given period of time T the frequency reaches linearly the value of 6 kHz to then return (in a time period much shorter than T - ideally null) to 100 Hz, by repeating such an operation a sound wave is obtained whose frequency is a (sawtooth) function with T period.

According to a particular aspect of the present invention, the T period of the sawtooth function is comprised between 5 and 20 seconds, preferably 10 seconds.

The Applicant noted that the stimulation of a culture of microorganisms (for example micro-algae) by means of a sound wave with frequency changing over time, as afore described, produces a growth increase with respect to the values obtained with sound waves having fixed frequency.

Such a result can be justified in that a modulated discontinuous pressure is probably better tolerated than a continuous and constant over time action. The biochemical response of compression and rarefaction in the frequencies of the sound wave allows the cells to somehow enjoy a healthy relaxing effect that is more effective in keeping the physiological balance of microorganisms (for example micro-algae).

According to a further aspect of the present invention, the intensity of the sound wave changes over time from 0 dB to 70 dB as a sawtooth function having the same period T. Preferably, the intensity and the frequency of the sound wave can change over time as sawtooth functions synchronized one to another and having the same period T (comprised between 5 and 20 seconds, preferably 10 seconds). Thus an increase in frequency corresponds to an increase in the sound wave intensity.

According to a further aspect of the present invention, the method comprises the step of c) detecting, by a hydrophone placed at least partially inside the culture of microorganisms, the signal irradiated by said sound wave inside said culture of microorganisms and the step of d) determining the concentration of the liquid culture on the basis of the signal detected by the hydrophone.

In particular, the step d) comprises the step of d1) calculating the Fourier transform of the sound wave detected during the step c), and the step of d2) calculating the acoustic pressure of the sound wave detected during the step c).

Thus, by means of an analysis in the frequency domain of the sound wave detected by the hydrophone, the Applicant observed that the sound wave crossing the culture has information on the number of cells of the microorganisms in the culture. In particular, the acoustic pressure measured through the hydrophone is proportional to the concentration of culture microorganisms changing over time. Thanks to the present invention the measurement of the number of cells per ml and the growth rate of microorganisms in the bioreactor can be obtained in continuous and automatically over time.

According to an advantageous aspect of the present invention the step a) comprises the step of a1) moving, through the shaking and mixing means, the culture of microorganisms with a reciprocating motion and along a substantially vertical direction.

Preferably the step a1) lasts about 60 secs and is repeated every 10 minutes.

According to the present invention a bioreactor is further implemented for producing a culture of microorganisms, comprising at least one cylindrical container (preferably having elliptical section) and shaking and mixing means to shake and mix the culture of microorganisms contained in the container.

According to an aspect of the present invention, the bioreactor further comprises a source of sound waves to irradiate the culture of microorganisms with a sound wave. In particular, the source is adapted to emit a sound wave whose frequency changes over time as a sawtooth function in a range comprised between 50 Hz and 8 kHz, preferably between 100 Hz and 6 kHz.

According to a further aspect of the present invention, the period T of the sawtooth function is comprised between 5 and 20 sec., preferably 10 sec. Preferably the intensity of the sound wave changes from 0 dB to 70 dB, at least during said period T.

According to a further aspect of the present invention, the bioreactor comprises at least one hydrophone placed at least partially inside the culture of microorganisms, to detect the signal irradiated by the source of sound wave inside the culture of microorganisms. As explained above, the signal detected by the hydrophone is used to determine the concentration of microorganisms of the culture.

Advantageously, the shaking and mixing means of the bioreactor according to the present invention comprise at least one shaking element provided with a rod having a substantially-vertical longitudinal axis and at least one first disk arranged obliquely with respect to the longitudinal axis of the rod.

Preferably, the shaking and mixing means comprise at least one second disk arranged obliquely with respect to the longitudinal axis of the rod. Preferably, said at least one first disk and said at least one second disk are arranged with opposed angles. Advantageously, the shaking and mixing means further comprise an electric motor to move the shaking element with a reciprocating motion in the direction of the longitudinal axis of the rod. The microorganisms can be thereby distributed continuously and homogeneously within the bioreactor, therefore preventing the formation of microorganism biofilms on the bioreactor walls and not interfering with the sound waves.

### Brief Description of the Drawings

Further aspects and advantages of the present invention will be more evident from the following description, made for illustration purposes only and without limitation, referring to the accompanying schematic drawings, in which:
- Figure 1 is an operating diagram of an embodiment of the bioreactor according to the present invention;
- Figures 2A and 2B are plots respectively of frequency and intensity of the irradiated sound wave versus time in an embodiment of the present invention;
- Figure 3 is an operating diagram of the shaking and mixing means according to the present invention;
- Figure 4 is a logarithmic scale spectrogram in T period of a sound signal used to generate the sound wave in an embodiment of the present invention.

### Embodiments of the invention

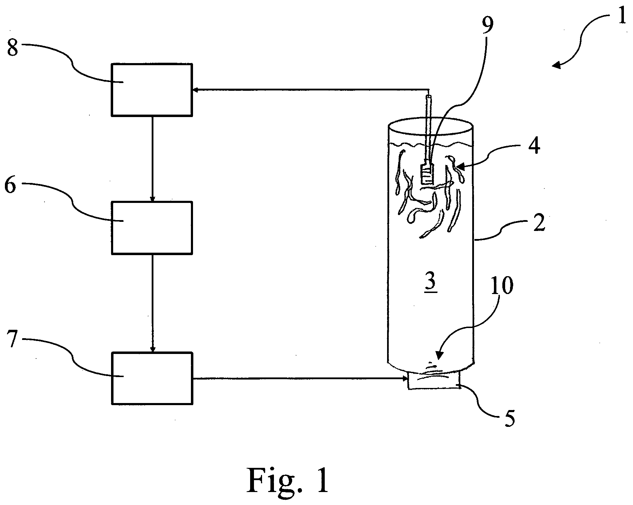

In figure 1 the operating diagram of a bioreactor 1 for producing a culture of microorganisms according to the present invention is shown. In the particular embodiment shown in figures, the bioreactor 1 has been used to produce a culture of micro-algae (for example *Scenedesmus obliquus*). Among the microalgae strains suitable for the use in the present invention, the following can be included by way of example and without limitation: *Scenedesmus obliquuus, Scenedesmus quadricaula, Scenedesmus sp., Chlorella emersonii, Chlorella vulgaris, Chlorella pyrenoidosa, Chlorella sorokiniana, Chlorella ellipsoidea, Chlorella salina, Chlorella minutissima, Chlorella protothecoides, Phaeodactulum tricornutum, Phaeodactylum tricornutum, Porphyridium cruentum, Spirulina platensis, Dunaliella salina, Dunaliella tertiolecta, Nannochloropsis salina, Nannochloropsis oculata, Nannochloropsis sp., Tetraselmis suecica, Tetraselmis chuii, Nannochloropsis Gaditana, Gymnodinium nelsoni, Isochrysis galbana, Euglena gracilis, Botrycoccus braunii, Neochloris oleoabundans, Chroomonas salina, Cyclotella cryptica, Nitzschia laevis, Onoraphidium sp., Haematococcus pluvialis, Monoraphidium minutum, Monoraphidium sp., Cyclotella sp., Ettlia texensis, Pavlova lutheri, Thalassiosira sp., Bacillaris stichococcus,* Microalgae cultured in monoculture or in competition among different species. Cultures of different species of cyanobacteria can further be used: *Anabaena sp, Anabaena variabilis, Arthrospira platensis, Arthrospira sp., Acaryochloris sp., Cyanothece sp., Microcystis aeruginosa, Microcystis sp., Rhodopseudomonas sp., Rhodopseudomonas palustris, Prochlorococcus marinus, Synechococcus sp., Synechococcus allunga, Nostoc sp., Nostoc punctiforme, Thermosynechococcus allunga, Trichodesmium erythraeum, Chlorobium tepidum, Chlorobaculum sp., Prochlorococcus sp., Acaryochloris marina, Gloeobacter sp., Gloeobacter violaceus, Synechocystis sp., Cylindrospermopsis raciborskii, Synechocystis sp.* Cultures of different species of diatoms can further be used: *Haslea ostrearia, Navicula ostrearia, Navicula sp. as water bioindicators, Licmophora ehrenbergii, Navicula tripunctata, Didimosphenia geminata, Cymbella sp., and exemplary yeasts: Saccharomyces cerevisiae, Saccharomyces kudriavzevii. Saccharomyces cariocanus, Saccharomyces paradoxus, Saccharomyces pastorianus, Saccharomyces bayanus, Saccharomyces monacensis, Saccharomyces uvarum. Saccharomyces carlsbergensis.*

The bioreactor 1 comprises a cylindrical container 2 adapted to contain the culture of microorganisms. In case of micro algae, the bioreactor further comprises one or more sources of light radiation (not shown), for example neon tubes on ceiling lamp arranged vertically at a given spacing, preferably 25 cm off the container. The container 2 is preferably made of 5 mm thick borosilicate glass.

Preferably the container has elliptical section with minor diameter equal to the radius of an equivalent tube. Such a characteristic allows distributing the volume of the microalgae cultures along the whole axis of the light source and, by exposing the surface with smaller curvature to light radiations, reflection phenomena are canceled out, limiting this dispersive effect just in proximity of the side profiles (more curved).

The container 2 of figure 1 is generally filled with doubly distilled water 3 in which a culture of microorganisms 4 is submerged (in this case microalgae). In proximity of the container base there is a sound source 5 (an acoustic actuator) to irradiate and stimulate the culture of microorganisms 4 with a sound wave 10. Preferably, the sound source 5 externally contacts the bottom wall of the container 2.

The Applicant further noted that, by means of LVD (Laser Doppler Vibrometer) laser interferometry analysis, in proximity of the respective outer profiles, the elliptic section of the container 2 provides the bioreactor with higher stiffness than in the steps of transmission of the sound waves, amplifies the oscillations themselves of the side surface of the container 2 more significantly than similar cylindrical structures in which, due to the rising of bothersome radial motions, the transmission effect of the sound vibrations is canceled out.

Additional embodiments of the present invention can however provide for the sound source being placed inside the container 2 and submerged in the doubly distilled water, still remaining in the protection scope of the present invention.

The sound source 5 can be an acoustic actuator (for example the Ottone™ actuator) with magnetostrictive technology, 20 W maximum power and 4 ohm impedance.

The sound source 5 is preferably connected to an amplifier 7 in turn connected to a signal generator 6. The signal generator 6 is preferably connected to an electronic processor 8 by which a given type of electrical signal can be uploaded to be generated by the generator 6, otherwise generator and electronic processor can be considered as a single operative unit (for example as a PC with a sound card). By means of the electronic processor and/or the generator a given electrical signal can be thus generated and, after having been conveniently amplified by the amplifier 7, is converted in a sound wave 10 by the sound actuator 5.

In a preferred embodiment, the bioreactor further comprises a hydrophone 9 submerged at least partially inside the culture 4 (preferably about 5-10 cm from the open water surface). The sound wave generated by the actuator 5 crosses the culture 4 and is then detected by the hydrophone 9. The hydrophone 9 is thus connected to an electronic processor 8 (for example by an acquisition card connected to a PC). After crossing the culture 4, the sound wave 10 contains some useful information that can be extracted by a frequency analysis of the signal detected by the hydrophone 9. The electronic processor 8 calculates the Fourier transform (for example by a FFT algorithm) of the signal detected by the hydrophone 9 and the acoustic pressure of the detected sound wave. On the basis of these data the processor 8 determines the concentration of microorganisms contained inside the culture 4.

For the intensity measurements of the sound wave in water, the unit of measure obtained after a series of conversions accounting for the temperature and the medium characteristics is the dB re 1 mPa.

In particular, by means of experimental tests a database can be realized wherein, per every concentration of microorganisms (measured by known techniques), the data related to a series of sample sounds (with a determined intensity and frequency) are associated. The concentration (cells/ml) measurements have been carried out with the laser grain size technique by Accusizer 780/AD Autodiluter after determining the diameter of the equivalent sphere projected on the optical path of the instrument while cells were crossing (that in the case of the Scenedesmus obliquus is the prolate spheroid) and the automatic cell counter with disposable tips Scepter™ 2.0 Handheld Automated Cell Counter.

By comparing the data related to the sound wave detected with the data contained in the database, the processor can accurately trace the concentration of microorganisms in the container. Such a measurement can therefore be done real time in order to keep the growth of the culture of microorganisms 4 monitored during the production cycle.

Preferably, the monitoring of the dimensional growth is carried out per classes 1-3 µm, 3-7 µm, 7-10 µm, 10-13 µm, rather than counting the cells in Burker chamber without appreciating the surface to volume ratio thereof.

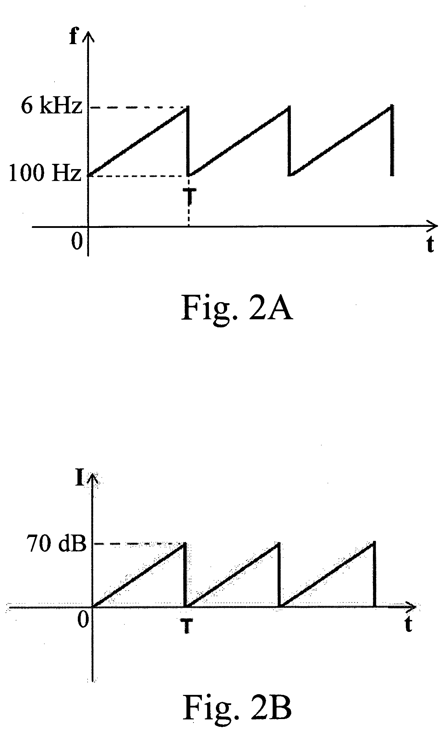

Preferably, the generated sound wave 10 (for example sinusoidal) has a frequency changing over time as a sawtooth function. In figure 2A the time pattern of the frequency of the sound wave 10 is shown. The frequency of the sound wave changes in a range comprised between 50 Hz and 8 kHz, preferably between 100 Hz and 6 kHz. The sawtooth function has a period T preferably comprised between 5 and 20 seconds, preferably 10 seconds.

Preferably, the intensity of the sound wave changes over time as a sawtooth function in a range comprised between 0 and 70 dB. In figure 2B the time pattern of the sound intensity (expressed in decibel) of the sound wave 10 generated by the actuator 5 is shown. In the shown embodiment the intensity (I) and the frequency (f) change over time synchronously in accordance with a sawtooth function with the same period T. Further embodiments can however provide for the intensity of the sound wave being maintained constant (for example at a value of about 62 dB) and for changing only the frequency, still remaining in the protection scope of the present invention.

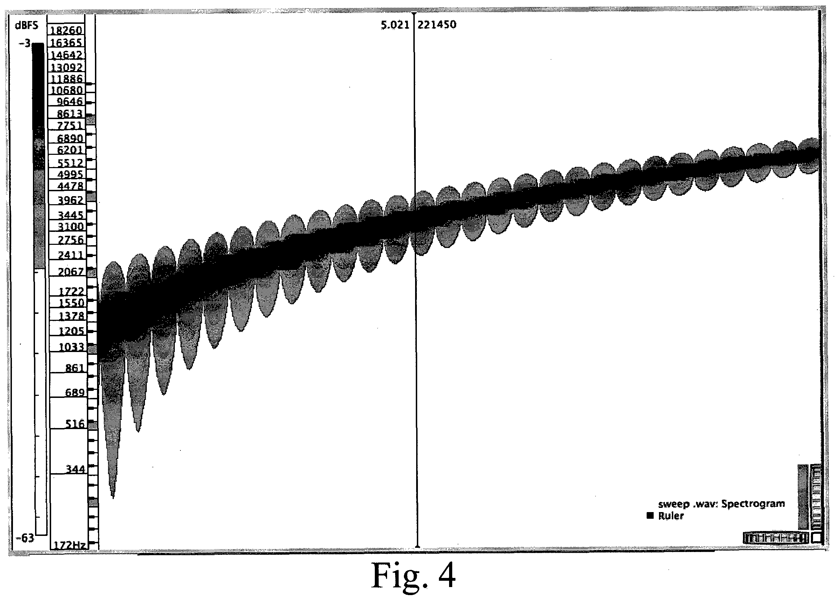

Figure 4 shows in greater detail the logarithmic scale spectrogram in T period of ten seconds of a sound signal used to generate the sound wave in a preferred embodiment of the present invention.

In case the microorganism growth needs artificial lighting (for example in case of microalgae), the culture is preferably cyclically lighted for 14 hours, in a period of 24 hours, with 10 hours dark. The acoustic stimulation is preferably carried out only during the fourteen light hours. More preferably, the acoustic stimulation is carried out in sessions of 40 minutes interspaced by a standby period of 140 minutes. In other words, sessions of acoustic stimulation lasting 40 minutes and "silence" sessions lasting 140 minutes are carried out alternately.

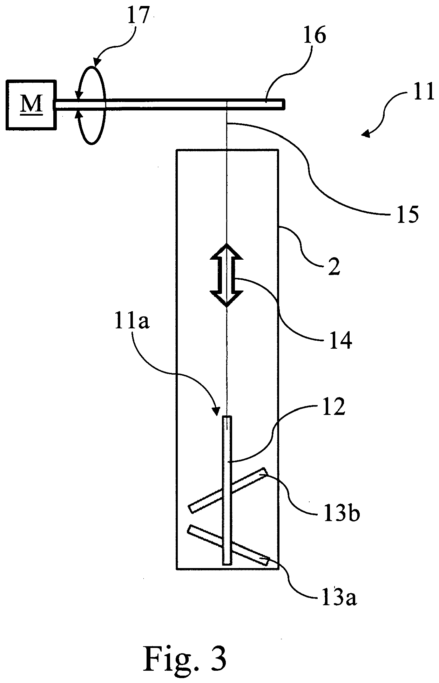

The bioreactor 1 further comprises means 11 for shaking and mixing the culture 4 of microorganisms contained in the container 2. In figure 1 for a better visualization the means 11 preferably have not been shown. In figure 3 an embodiment is shown of the means 11 for shaking and mixing the culture 4 of microorganisms according to the present invention.

The shaking and mixing means 11 comprise at least one shaking element 11a (located within the container 2) provided with a rod 12 having a substantially vertical longitudinal axis and at least one first disk 13a arranged obliquely with respect to the longitudinal axis of the rod 12. In the shown embodiment, the shaking element 11a is provided with two disks 13a, 13b fastened to the rod 12 and preferably arranged obliquely with respect to the longitudinal axis of the rod 12, more preferably arranged with opposed angles. The disks angle with respect to the bottom of the container 2 is preferably of about 20°.

Further embodiments can however provide for the rod 12 being provided with a larger number of disks (for example three or more disks) of which at least one disk is angularly oriented in opposition to the other disks, still remaining in the protection scope of the present invention.

The rod 12 is preferably made of steel. Preferably the disks 13a and 13b are made of Teflon (preferably with a thickness of about 4 mm) in order to ensure the sliding capability and to minimize possible frictions with the inner walls of the container 2. The shaking element 11a is therefore moved with a reciprocating motion and along a substantially vertical direction, as shown by the arrows 14 in figure 3. By moving the shaking element 11a within the container 2 with a reciprocating bottom-up motion and vice versa, the Applicant noted the triggering of rotation motions (i.e. micro-vortexes in absence of turbulence) by which a homogeneous distribution of the microorganisms within the culture 4 and a better resuspension of the microorganisms along the water column 3 are obtained.

The longitudinal reciprocating motion can be obtained for example by winding and unrolling a nylon thread 15 (fastened at an end of the rod 12) around an aluminium bar 16 placed horizontally above the container 2 and moved clockwise and counterclockwise (as shown by the arrows 17) by means of an electric motor M. The electric motor M is driven by alternating pulses (positive and negative) each lasting six seconds. The shaking element 11a is then moved so that a complete "up and down" cycle is completed in 12 seconds (six seconds for the rise and six seconds for the descent). Such an operation is repeated for a minute (five complete "up and down" cycles), every ten minutes, i.e. one minute of shaking and nine minutes of standby.

Preferably, in case of cultures 4 of microalgae, the steps of one minute shaking and nine minutes standby are repeated continuously both in the lightening period and in the dark period and, similarly, during the period of acoustic stimulation and the silence period.

The embodiment shown in figure 3 allows reducing the bulk of the means 11, additional embodiments can however provide for obtaining the longitudinal reciprocating motion of the shaking element 11a in different ways, for example the rod 12 can be moved by means of a rigid element, for example a rack (in place of the thread) coupled with a pinion rotated by the electric motor M.

An alternative embodiment provides for the bioreactor 1 comprising a plurality of containers 2, each of which is equipped with a source 5 of sound waves 10. In this embodiment each container 2 is provided with a shaking element 11a. Each shaking element 11a is moved by winding and unrolling a corresponding nylon thread 15 around a bar 16 rotated by the motor M. In this way, by means of a single electrical motor M, various shaking elements 11a can moved longitudinally and synchronously, thereby obtaining a simple and inexpensive to implement structure. In general, the method of producing a culture of microorganisms according to the present invention comprises the steps of:
a) shaking and mixing said microorganisms 4 through said shaking and mixing means 11,
b) irradiating said culture of microorganisms 4 with a sound wave 10 whose frequency (f) changes over time as a sawtooth function in a range comprised between 50 Hz and 8 kHz, preferably between 100 Hz and 6 kHz.
   Preferably the period T of the sawtooth function is comprised between 5 and 20 seconds, preferably 10 seconds.
   Preferably the method comprises the steps of:
c) detecting, by a hydrophone 9 placed at least partially inside said culture of microorganisms 4, said sound wave 10 crossing said culture of microorganisms 4,
d) determining the concentration of said culture 4 on the basis of the signal provided by said hydrophone 9.

Preferably, during the step b) the intensity (I) of said sound wave 10 changes over time as a sawtooth function in a range comprised between 0 dB to 70 dB.

The step a) comprises the step of a1) moving, by said shaking and mixing means 11, said culture of microorganisms 4 with a reciprocating motion and along a substantially vertical direction 14. Preferably the step a1) lasts about 60 sec and is repeated every 10 minutes.

Preferably the step d) comprises the step of d1) calculating the Fourier transform of the sound wave 10 detected during the step c), and the step of d2) calculating the acoustic pressure of the sound wave 10 detected during the step c).

## Claims

1. Method of producing a culture of microorganisms (4) by means of a bioreactor (1) comprising at least one cylindrical container (2) and shaking and mixing means (11) to shake and mix said culture of microorganisms (4) contained in said container (2), said method comprising the step of a) shaking and mixing said microorganisms (4) by said shaking and mixing means (11), and the step of b) irradiating said culture of microorganisms (4) with a sound wave (10), **characterized in that** said step b) comprises the step of irradiating said culture (4) by a sound wave (10) whose frequency (f) changes over time as a sawtooth function in a range comprised between 50 Hz and 8 kHz, preferably between 100 Hz and 6 kHz.

2. Method according to claim 1, **characterized in that** the intensity (I) of said sound wave (10) changes over time as a sawtooth function in a range comprised between 0 dB to 70 dB.

3. Method according to claim 1 or 2, **characterized in that** the period (T) of said sawtooth function is comprised between 5 and 20 sec., preferably 10 sec.

4. Method according to one or more of the preceding claims, **characterized by** comprising the step of c) detecting by a hydrophone (9) placed at least partially inside said culture of microorganisms (4), said sound wave (10) crossing said culture of microorganisms (4) and the step of d) determining the concentration of said culture (4) on the basis of the signal provided by said hydrophone (9).

5. Method according to claim 4, **characterized in that** said step d) comprises the step of d1) calculating the Fourier transform of the signal of the sound wave (10) detected during the step c), and the step of d2) calculating the acoustic pressure of the sound wave (10) detected during the step c).

6. Method according to one or more of the preceding claims, **characterized in that** said step a) comprises the step of a1) moving, through said shaking means (11) said culture of microorganisms (4) with a reciprocating motion and along a substantially vertical direction (14).

7. Method according to claim 6, **characterized in that** said step a1) lasts about 60 sec and is repeated every 10 minutes.

8. Bioreactor (1) for producing a culture of microorganisms (4) comprising at least one cylindrical container (2) and shaking and mixing means (11) to shake and mix said culture of microorganisms (4) contained in said container (2), said bioreactor (1) further comprising a source of sound waves (5) to irradiate said culture of microorganisms (4) with a sound wave (10), **characterized in that** said source (5) is adapted to emit a sound wave (10) whose frequency (f) changes over time as a sawtooth function in a range comprised between 50 Hz and 8 kHz, preferably between 100 Hz and 6 kHz.

9. Bioreactor (1) according to claim 8, **characterized in that** the period (T) of said sawtooth function is comprised between 5 and 20 sec., preferably 10 sec, and **in that** the intensity of said sound wave (10) changes from 0 dB to 70 dB at least during said period (T).

10. Bioreactor (1) according to claim 8 or 9, **characterized by** comprising at least one hydrophone (9), placed at least partially inside said culture of microorganisms (4), to detect the sound wave irradiated from said source (5) and crossing said culture of microorganisms (4), said sound wave detected by said hydrophone (9) being used to determine the concentration of said liquid culture (4).

11. Bioreactor (1) according to one or more of claims 8 to 10, **characterized in that** said shaking and mixing means (11) comprise at least one shaking element (11a) provided with a rod (12) having a substantially-vertical longitudinal axis and at least one first disk (13a) arranged obliquely with respect to said longitudinal axis of said rod (12).

12. Bioreactor (1) according to claim 11, **characterized in that** said shaking and mixing means (11) comprise at least one second disk (13b) arranged obliquely with respect to said longitudinal axis of said rod (12), said at least one first disk (13a) and said at least one second disk (13b) being arranged with opposed angles.

13. Bioreactor (1) according to claim 11 or 12, **characterized in that** said shaking and mixing means (11) further comprise an electric motor (M) to move said shaking element (11a) with a reciprocating motion in the direction (14) of said longitudinal axis of said rod (12).

## Patentansprüche

1. Verfahren zur Herstellung einer Kultur von Mikroorganismen (4) mittels eines Bioreaktors (1), umfassend mindestens einen zylindrischen Behälter (2) und Schüttel- und Mischmittel (11) zum Schütteln und Mischen der in dem Behälter (2) enthaltenen Kultur von Mikroorganismen (4), wobei das Verfahren den Schritt umfasst: a) Schütteln und Mischen der Mikroorganismen (4) durch die Schüttel- und Mischeinrichtung (11) und den Schritt umfasst: b) Beschallen der Kultur von Mikroorganismen (4) mit einer Schallwelle (10), **dadurch gekennzeichnet, dass** der Schritt b) den Schritt des Beschallens der Kultur (4) mit einer Schallwelle (10) umfasst, deren Frequenz (f) sich im Laufe der Zeit als eine Sägezahnfunktion in einem Bereich zwischen 50 Hz und 8 kHz, vorzugsweise zwischen 100 Hz und 6 kHz, verändert.

2. Verfahren nach Anspruch 1, **dadurch gekennzeichnet, dass** die Intensität (I) der Schallwelle (10) sich im Laufe der Zeit als eine Sägezahnfunktion in einem Bereich zwischen 0 dB und 70 dB verändert.

3. Verfahren nach Anspruch 1 oder 2, **dadurch gekennzeichnet, dass** die Periode (T) der Sägezahnfunktion zwischen 5 und 20 Sekunden, vorzugsweise 10 Sekunden, liegt.

4. Verfahren nach einem oder mehreren der vorhergehenden Ansprüche, **dadurch gekennzeichnet, dass** es den Schritt umfasst: c) Erfassen durch ein Hydrophon (9), das zumindest teilweise innerhalb der Kultur von Mikroorganismen (4) angeordnet ist, der Schallwelle (10), die die Kultur von Mikroorganismen (4) kreuzt, und den Schritt umfasst: d) Bestimmen der Konzentration der Kultur (4) auf der Grundlage des Signals, das von dem Hydrophon (9) geliefert wird.

5. Verfahren nach Anspruch 4, **dadurch gekennzeichnet, dass** der Schritt d) den Schritt d1) des Berechnens der Fourier-Transformation des während des Schrittes c) erfassten Signals der Schallwelle (10) umfasst und den Schritt d2) des Berechnens des Schalldrucks der in Schritt c) erfassten Schallwelle (10) umfasst.

6. Verfahren nach einem oder mehreren der vorhergehenden Ansprüche, **dadurch gekennzeichnet, dass** der Schritt a) den Schritt umfasst: a1) Bewegen der Kultur von Mikroorganismen (4) durch die Schütteleinrichtung (11) in einer Hin- und Herbewegung und entlang einer im Wesentlichen vertikalen Richtung (14).

7. Verfahren nach Anspruch 6, **dadurch gekennzeichnet, dass** der Schritt a1) etwa 60 Sekunden andauert und alle 10 Minuten wiederholt wird.

8. Bioreaktor (1) zur Herstellung einer Kultur von Mikroorganismen (4), umfassend mindestens einen zylindrischen Behälter (2) und Schüttel- und Mischmittel (11) zum Schütteln und Mischen der in dem Behälter (2) enthaltenen Kultur von Mikroorganismen (4), wobei der Bioreaktor (1) ferner eine Schallwellenquelle (5) aufweist, um die Kultur von Mikroorganismen (4) mit einer Schallwelle (10) zu beschallen, **dadurch gekennzeichnet, dass** die Quelle (5) dazu eingerichtet ist, eine Schallwelle (10) zu emittieren, deren Frequenz (f) sich im Laufe der Zeit als eine Sägezahnfunktion in einem Bereich zwischen 50 Hz und 8 kHz, vorzugsweise zwischen 100 Hz und 6 kHz, verändert.

9. Bioreaktor (1) nach Anspruch 8, **dadurch gekennzeichnet, dass** die Periode (T) der Sägezahnfunktion zwischen 5 und 20 Sekunden, vorzugsweise 10 Sekunden, liegt und dass sich die Intensität der Schallwelle (10) von 0 dB bis 70 dB zumindest während der Periode (T) verändert.

10. Bioreaktor (1) nach Anspruch 8 oder 9, **dadurch gekennzeichnet, dass** dieser mindestens ein Hydrophon (9) umfasst, das zumindest teilweise innerhalb der Kultur von Mikroorganismen (4) angeordnet ist, um die von der Quelle (5) ausgesendete und die Kultur von Mikroorganismen (4) kreuzende Schallwelle zu erfassen, wobei die durch das Hydrophon (9) erfasste Schallwelle zur Bestimmung der Konzentration der Flüssigkultur (4) verwendet wird.

11. Bioreaktor (1) nach einem oder mehreren der Ansprüche 8 bis 10, **dadurch gekennzeichnet, dass** die Schüttel- und Mischmittel (11) mindestens ein Schüttelelement (11a) umfassen, das mit einer Stange (12) versehen ist, die eine im Wesentlichen vertikale Längsachse hat, und mindestens eine erste Scheibe (13a) umfassend, die schräg zur Längsachse der Stange (12) angeordnet ist.

12. Bioreaktor (1) nach Anspruch 11, **dadurch gekennzeichnet, dass** die Schüttel- und Mischmittel (11) mindestens eine zweite Scheibe (13b) umfassen, die schräg in Bezug auf die Längsachse der Stange (12) angeordnet ist, wobei die erste Scheibe (13a) und die mindestens eine zweite Scheibe (13b) mit entgegengesetzten Winkeln angeordnet sind.

13. Bioreaktor (1) nach Anspruch 1 1 oder 12, **dadurch gekennzeichnet, dass** die Schüttel- und Mischmittel (11) weiterhin einen Elektromotor (M) umfassen, um das Schüttelelement (11a) in einer Hin- und Herbewegung in der Richtung (14) der Längsachse der Stange (12) zu bewegen.

## Revendications

1. Procédé de production d'une culture de microorganismes (4) au moyen d'un biréacteur (1) comprenant au moins un réceptacle cylindrique (2) et des moyens d'agitation et de mélange (11) pour agiter et mélanger ladite culture de microorganismes (4) contenue dans ledit réceptacle (2), ledit procédé comprenant l'étape a) d'agitation et de mélange desdits microorganismes (4) par lesdits moyens d'agitation et de mélange (11) et l'étape b) d'irradiation de ladite culture de microorganismes (4) par une onde sonore (10), **caractérisé en ce que** ladite étape b) comprend l'étape d'irradiation de ladite culture (4) par une onde sonore (10) dont la fréquence (f) varie au fil du temps comme une fonction en dents de scie dans une plage comprise entre 50 Hz et 8 kHz, de préférence entre 100 Hz et 6 kHz.

2. Procédé selon la revendication 1, **caractérisé en ce que** l'intensité (i) de ladite onde sonore (10) varie au fil du temps comme une fonction en dents de scie dans une plage comprise entre 0 dB et 70 dB.

3. Procédé selon la revendication 1 ou 2, **caractérisé en ce que** la période (t) de ladite fonction en dents de scie est comprise entre 5 et 20 s, de préférence de 10 s.

4. Procédé selon une ou plusieurs des revendications précédentes, **caractérisé en ce qu'**il comprend l'étape c) de détection par un hydrophone (9) placé au moins en partie à l'intérieur de ladite culture de microorganismes (4), ladite onde sonore (10) traversant ladite culture de microorganismes (4), et l'étape d) de détermination de la concentration de ladite culture (4) sur la base du signal fourni par ledit hydrophone (9).

5. Procédé selon la revendication 4, **caractérisé en ce que** ladite étape d) comprend l'étape d1) de calcul de la transformée de Fourier du signal de l'onde sonore (10) détectée au cours de l'étape c), et l'étape d2) de calcul de la pression acoustique de l'onde sonore (10) détectée au cours de l'étape c).

6. Procédé selon une ou plusieurs des revendications précédentes, **caractérisé en ce que** ladite étape a) comprend l'étape a1) de déplacement à travers lesdits moyens d'agitation (11) de ladite culture de microorganismes (4) avec un mouvement de va-et-vient et le long d'une direction sensiblement verticale (14).

7. Procédé selon la revendication 6, **caractérisé en ce que** ladite étape a1) dure environ 60 s et est répétée toutes les 10 minutes.

8. Bioréacteur (1) pour produire une culture de microorganismes (4) comprenant au moins un réceptacle cylindrique (2) et des moyens d'agitation et de mélange (11) pour agiter et mélanger ladite culture de microorganismes (4) contenue dans ledit réceptacle (2), ledit bioréacteur (1) comprenant en outre une source d'ondes sonores (5) pour irradier ladite culture de microorganismes (4) par une onde sonore (10), **caractérisé en ce que** ladite source (5) est à même d'émettre une onde sonore (10) dont la fréquence (f) varie au fil du temps comme une fonction en dents de scie dans une plage comprise entre 50 Hz et 8 kHz, de préférence entre 100 Hz et 6 kHz.

9. Bioréacteur (1) selon la revendication 8, **caractérisé en ce que** la période (T) de ladite fonction en dents de scie est comprise entre 5 et 20 s, de préférence de 10 s, et **en ce que** l'intensité de ladite onde sonore (10) varie de 0 dB à 70 dB au moins au cours de ladite période (T).

10. Bioréacteur (1) selon la revendication 8 ou 9, **caractérisé en ce qu'**il comprend au moins un hydrophone (9), placé au moins en partie à l'intérieur de ladite culture de microorganismes (4) pour détecter l'onde sonore irradiée par ladite source (5) et traversant ladite culture de microorganismes (4), ladite onde sonore détectée par ledit hydrophone (9) étant utilisée pour déterminer la concentration de ladite culture liquide (4).

11. Bioréacteur (1) selon une ou plusieurs des revendications 8 à 10, **caractérisé en ce que** lesdits moyens d'agitation et de mélange (11) comprennent au moins un élément d'agitation (11a) pourvu d'une tige (12) ayant un axe longitudinal sensiblement vertical et au moins un premier disque (13a) agencé en oblique par rapport audit axe longitudinal de ladite tige (12).

12. Bioréacteur (1) selon la revendication 11, **caractérisé en ce que** lesdits moyens d'agitation et de mélange (11) comprennent au moins un second disque (13b) agencé en oblique par rapport audit axe longitudinal de ladite tige (12), ledit au moins un premier disque (13a) et ledit au moins un second disque (13b) étant agencés avec des angles opposés.

13. Bioréacteur (1) selon la revendication 11 ou 12, **caractérisé en ce que** lesdits moyens d'agitation et de mélange (11) comprennent en outre un moteur électrique (M) pour déplacer ledit élément d'agitation (11a) avec un mouvement de va-et-vient dans la direction (14) dudit axe longitudinal de ladite tige (12).
